# EUROPEAN PATENT APPLICATION

(11) **EP 4 667 924 A1**
(43) Date of publication of application: **24.12.2025**
(21) Application number: 23922828.1
(22) Date of filing: 31.08.2023
(51) Int. Cl.: G01N 27/327, G01N 27/416, G01N 33/53, G01N 33/543, G01N 33/566

(54) **ELECTROCHEMICAL ANALYSIS KIT, ELECTROCHEMICAL ANALYSIS SYSTEM, AND ELECTROCHEMICAL ANALYSIS METHOD**

(30) Priority: 14.02.2023 JP 2023021210
(71) Applicant: NEC Solution Innovators, Ltd., Koto-ku, Tokyo 136-8627 (JP); Immunosens Co., Ltd., Suita-shi, Osaka 564-8565 (JP)
(72) Inventor: HORII Katsunori, Tokyo 136-8627 (JP); MINAGAWA Hirotaka, Tokyo 136-8627 (JP); INAGUMA Asumi, Tokyo 136-8627 (JP); AKIYAMA Yu, Tokyo 136-8627 (JP); HIROSE Miwako, Tokyo 136-8627 (JP); FUJITA Tomoko, Tokyo 136-8627 (JP); YAMAMURA Koutarou, Kishibe-Shimmachi, Suita-shi, Osaka 564-8565 (JP); IDE Asuka, Kishibe-Shimmachi, Suita-shi, Osaka 564-8565 (JP)
(74) Representative: Betten & Resch
(86) International application number: PCT/JP2023/031836
(87) International publication number: WO 2024/171494

(57) **Abstract**

The electrochemical analysis kit includes an electrode section, an immobilized conjugate (2), and a labeled conjugate (4). The electrode section includes a pair of electrodes: a working electrode (11) and a counter electrode (12), the immobilized conjugate (2) specifically binding to an analyte (3), and is immobilized at a specific site in a state of being bound to the analyte (3), the labeled conjugate (4) also binding to the analyte (3), and is labeled with a metal particle (4a), when the sample contains the analyte (3), both conjugates (2, 4) bind to the analyte (3), causing the labeled conjugate (4) to be immobilized at a specific site, the specific site enables the metal particle (4a) to undergo oxidation, followed by reduction via potential control of the working electrode (11), resulting current is measured through the electrodes pair, and at least one of the conjugates (2, 4) is an aptamer.

## Description

### INCORPORATION BY REFERENCE

This application is based upon and claims the benefit of priority from Japanese patent application No. 2023-021210, filed on February 14, 2023, the disclosure of which is incorporated herein in its entirety by reference.

### TECHNICAL FIELD

The present invention relates to an electrochemical analysis kit, an electrochemical analysis system, and an electrochemical analysis method.

### BACKGROUND ART

As one of methods for analyzing trace substances in samples, an analysis method using antigen-antibody reaction is known (Patent Literature 1). The determination of the presence of an antigen described in Patent Literature 1 can be performed by forming an immune complex between an antigen in a sample and an antibody labeled with metal colloid or the like, and visually checking color developed as a result of the immune complex being captured on a capture antibody.

### Citation List

### Patent Literature

Patent literature 1: JP 2017-133857 A

### SUMMARY

### Technical Problem

The lateral flow test as described in Patent Literature 1 is suitable for qualitative analysis but is not very suitable for quantitative analysis. In addition, antibodies, which are proteins, concern problems with stability and the like.

In this regard, aptamers (nucleic acid molecules) have the function of specifically binding to antigens and are advantageous over antibodies in terms of stability and the like. In lateral flow tests, for example, as described in Patent Literature 1, there are few examples using aptamers, while antigen-antibody reaction is mainly utilized.

Therefore, an example object of the invention is to provide a new electrochemical analysis kit that uses aptamer, and is capable of quantitative analysis, an electrochemical analysis system including the analysis kit, and an electrochemical analysis method using the analysis kit.

### Solution to Problem

An electrochemical analysis kit according to an example aspect of the invention includes an electrode section, an immobilized conjugate, and a labeled conjugate, wherein the electrode section includes a pair of electrodes, the pair of electrodes includes a working electrode, and a counter electrode, the immobilized conjugate is capable of specifically binding to an analyte, and of being immobilized at a specific site in a state of being bound to the analyte, the labeled conjugate is capable of specifically binding to the analyte, and is labeled with a metal particle, when the analyte is contained in a sample to be analyzed, the immobilized conjugate and the labeled conjugate bind to the analyte, so that the labeled conjugate be immobilized at the specific site, the specific site is a position where the metal particle can be oxidized by control of potential of the working electrode, after the oxidation of the metal particle, the metal particle is reduced by control of potential of the working electrode, current generated by the reduction can be measured with the pair of electrodes, and a conjugate of at least one of the immobilized conjugate or the labeled conjugate is an aptamer.

An electrochemical analysis system according to an example aspect of the invention includes an electrochemical analysis apparatus, and an electrochemical analysis kit, wherein the electrochemical analysis kit is the kit according to an example aspect of the invention, and the electrochemical analysis apparatus is capable of applying voltage to the electrode and measuring current of the electrode.

An electrochemical analysis method according to an example aspect of the invention uses the kit according to an example aspect of the invention, and includes oxidizing the metal particle bound to the labeled conjugate by controlling the potential of the working electrode, reducing the metal particle by controlling the potential of the working electrode, and qualitatively or quantitatively analyzing the analyte by measuring the current generated by the reduction with the pair of electrodes.

### Advantageous Effects of Invention

An example advantage according to the invention is that a new electrochemical analysis kit that uses aptamer, and is capable of quantitative analysis, an electrochemical analysis apparatus, an electrochemical analysis system including the analysis kit, and an electrochemical analysis method using the analysis kit can be provided.

### BRIEF DESCRIPTION OF DRAWINGS

[FIG. 1] FIG. 1 is a schematic diagram showing an example of the electrochemical analysis method according to a first example embodiment.
[FIG. 2A] FIG. 2A is a schematic diagram showing an example of the electrochemical analysis method according to a second example embodiment.
[FIG. 2B] FIG. 2B is a schematic diagram showing an example of the electrochemical analysis method according to the second example embodiment.
[FIG. 3] FIG. 3 is a structural diagram showing an example of a lateral flow strip in the electrochemical analysis method according to a third example embodiment.
[FIG. 4] FIG. 4 is a schematic diagram showing an example of the electrochemical analysis method according to a third example embodiment.
[FIG. 5] FIG. 5 is a schematic diagram showing an example of the electrochemical analysis method according to a fourth example embodiment.
[FIG. 6A] FIG. 6A is a graph showing a relationship between sIgA concentration and sensor output in Example 1.
[FIG. 6B] FIG. 6B is another graph showing a relationship between sIgA concentration and sensor output in Example 1.
[FIG. 7] FIG. 7 is a graph showing a relationship between sensor output obtained from the electrochemical analysis kit of the invention and absorbance at a wavelength of 450 nm obtained by ELISA method in Example 2.

### EXAMPLE EMBODIMENTS

The example embodiments of the invention are described below with reference to the drawings. It is to be noted, however, that the invention is by no means limited or restricted by the following example embodiments. In the following drawings, identical parts are indicated with identical reference signs. Regarding the descriptions of the example embodiments, reference can be made to one another unless otherwise stated. Furthermore, the configurations of the example embodiments can be combined unless otherwise stated.

### [Electrochemical Analysis Kit]

First, the electrochemical analysis kit of the invention will be described. The electrochemical analysis kit of the invention (hereinafter also referred to as an "analysis kit") includes an electrode section, an immobilized conjugate, and a labeled conjugate.

The electrode section includes a pair of electrodes, and the pair of electrodes are a working electrode and a counter electrode. For example, in the pair of electrodes, the working electrode and the counter electrode are interchangeable with each other.

Materials for the working electrode and the counter electrode are not particularly limited, and examples thereof include conductive materials such as metal and conductive carbon. For example, the conductive material itself may be the working electrode or the counter electrode, or the conductive material may be applied or laminated on a support or the like to form the working electrode and the counter electrode. In the case where the conductive material is applied, for example, a solution of the conductive material may be applied by printing or the like. In the case where the conductive material is laminated, for example, the conductive material may be formed into a film and laminated via a pressure-sensitive adhesive or the like. In the case of applying the conductive carbon, examples of the conductive carbon include ketjen black, graphene, carbon nanotubes, and fullerenes.

The electrode section further includes, for example, a reference electrode. The reference electrode is not particularly limited as long as, for example, having stability and reproducibility of electrode potential, and examples thereof include silver/silver chloride. The reference electrode may be provided by, for example, applying the silver/silver chloride or the like on the conductive material or the like. In the case where the conductive material is applied, for example, a solution of the conductive material may be applied by printing or the like. In the case of applying the silver/silver chloride, the silver/silver chloride may be, for example, a paste including fine particles of silver chloride crystals.

The electrode section may be covered with, for example, an electrical insulator. Examples of the electrical insulator include polymers such as urethane resins, polyamide resins, and phenol resins.

The immobilized conjugate is capable of specifically binding to an analyte, and of being immobilized at a specific site in a state of being bound to the analyte. The analyte is, for example, a biomarker. The biomarker is, for example, secretory immunoglobulin A (sIgA). The immobilization is not particularly limited, and examples thereof include immobilization by chemical bonds such as covalent bonds, intermolecular interactions, and ionic bonds, and immobilization by magnetic force.

In the case of the immobilization by magnetic force, the immobilized conjugate may be, for example, one that is capable of specifically binding to the analyte and is labeled with a magnetic substance such as a magnetic particle. In this case, when the analyte is contained in a sample to be analyzed, the immobilized conjugate labeled with the magnetic particle, and the labeled conjugate bind to the analyte. As a result, the labeled conjugate binds to the immobilized conjugate via the analyte.

The labeled conjugate is capable of specifically binding to the analyte and is labeled with a metal particle. When the analyte is contained in a sample to be analyzed, the immobilized conjugate and the labeled conjugate bind to the analyte, and thereby the labeled conjugate can be immobilized at the specific site. The specific site is a site where the metal particle can be oxidized by the control of the potential of the working electrode.

The metal particle is not particularly limited, and examples thereof include metal colloids. The metal particle is, for example, a metal particle capable of being electrolyzed, and is capable of measuring current generated by reduction described below. Examples of the metal include gold, platinum, silver, copper, rhodium, and palladium. The weight-average particle size of the metal particle is not particularly limited, and is, for example, 20 nm or more, or 40 nm or more, and 100 nm or less, or 60 nm or less. A method for the labeling with the metal particle is not particularly limited and may be, for example, labeling using the chemical bonds as described above.

After oxidizing the metal particle, the metal particle is reduced by controlling the potential of the working electrode, and current generated by the reduction can be measured with the pair of electrodes.

A conjugate of at least one of the immobilized conjugate or the labeled conjugate is an aptamer. Also, for example, a conjugate of one of the immobilized conjugate or the labeled conjugate may be an antibody. The aptamer is not particularly limited as long as being capable of, for example, specifically binding to the analyte, and being immobilized at a specific site in a state of being bound to the analyte. When the analyte is sIgA, for example, the sIgA-binding nucleic acid molecule described in Japanese Patent No. 6795211, or other sIgA-binding nucleic acid molecules may be used.

The electrochemical analysis kit of the invention may further include, for example, a lateral flow analysis section. The lateral flow analysis section includes, for example, a development region, a sample supply region, and a determination region. For example, the sample supply region is arranged at one end of the development region, and the determination region is arranged at the opposite side of the development region from the sample supply region. At the determination region, for example, the electrode section and the immobilized conjugate are arranged. The sample to be analyzed and the labeled conjugate can be supplied to the sample supply region. For example, in the development region, the sample and the labeled conjugate can move to the determination region by capillary phenomenon.

In the lateral flow analysis section, for example, the development region, the sample supply region, the determination region and the like may be provided on a support or the like. The support is not particularly limited and may be, for example, a resin support.

The development region may be, for example, a fiber pad used in a conventional lateral flow test kit. The fiber pad is, for example, a nonwoven fabric formed of fibers. The nonwoven fabric may be, for example, any nonwoven fabric as long as having capillary force formed of glass fibers, resin fibers, carbon fibers, natural fibers, or the like. The thickness of the nonwoven fabric is not particularly limited, and is, for example, 0.3 to 1.0 mm.

The lateral flow analysis section may further include, for example, a movement control region. The movement control region controls, for example, the movement of the sample and the labeled aptamer developed in the development region to the determination region. The control of the movement includes, for example, control of the movement speed, the movement amount (flow amount), and the like. The control of the movement is performed by, for example, external action on the movement control part, or the like. The external action is, for example, pressing pressure or the like. The method for applying the pressing pressure is not particularly limited and may be, for example, providing a projection or the like on a back surface of a case housing the lateral flow test kit of the invention, and applying the pressing pressure to the movement control region by the projection or the like. A position to which the pressing pressure is applied in the movement control region is not particularly limited and may be, for example, one or more positions, or two or more positions. The movement control region controls the movement speed of the sample and the labeled aptamer, and thereby, for example, securing time for reaction between the sample and the labeled aptamer.

The movement control region is, for example, a nonwoven fabric formed of fibers. The nonwoven fabric may be, for example, any nonwoven fabric as long as having capillary force formed of glass fibers, resin fibers, carbon fibers, natural fibers, or the like. The thickness of the nonwoven fabric is not particularly limited and is, for example, 0.3 to 1.0 mm, or 0.1 to 0.7 mm.

### [Electrochemical Analysis System]

Next, the electrochemical analysis system of the invention will be described. The electrochemical analysis system of the invention (hereinafter also referred to as an "analysis system") includes an electrochemical analysis apparatus and an electrochemical analysis kit. The electrochemical analysis kit is the electrochemical analysis kit of the invention.

The electrochemical analysis apparatus is capable of applying voltage to the electrode and measuring the current of the electrode. Therefore, the electrochemical analysis apparatus is not particularly limited as long as being capable of applying voltage to the electrode and measuring the current of the electrode. By applying voltage to the electrode, for example, the metal particles are oxidized. When the metal particles are oxidized, the current value in the electrode changes. Therefore, for example, by measuring the current of the electrode, the presence of the analyte or the concentration of the analyte can be measured.

The electrochemical analysis system of the invention allows, for example, determination of the immune state of a human by measuring the concentration of sIgA contained in saliva. In this respect, for example, measurement data of the sIgA concentration may be transmitted to a terminal capable of processing the measurement data to determine the immune state of a human based on the measurement data by the terminal.

The electrochemical analysis system of the invention can easily realize the oxidation of metal fine particles by the control of the potential of the working electrode. Therefore, it is possible to avoid complication of the measurement operation to the maximum extent possible, compared to, for example, oxidation by chemical treatment or the like. In addition, highly sensitive and accurate measurement of a test substance in a test solution can be realized by simple operation without requiring large-sized measuring instruments.

### [Electrochemical Analysis Method]

Next, the electrochemical analysis method of the invention will be described. The electrochemical analysis method of the invention (hereinafter also referred to as an "analysis method") uses the electrochemical analysis kit of the invention, and includes processes of oxidizing, reducing, and analyzing. The oxidizing is a process of controlling the potential of the working electrode to oxidize the metal particles bound to the labeled conjugates. The reducing is a process of controlling the potential of the working electrode to reduce the metal particles. The analyzing is a process of measuring the current generated by the reduction to qualitatively or quantitatively analyze the analyte.

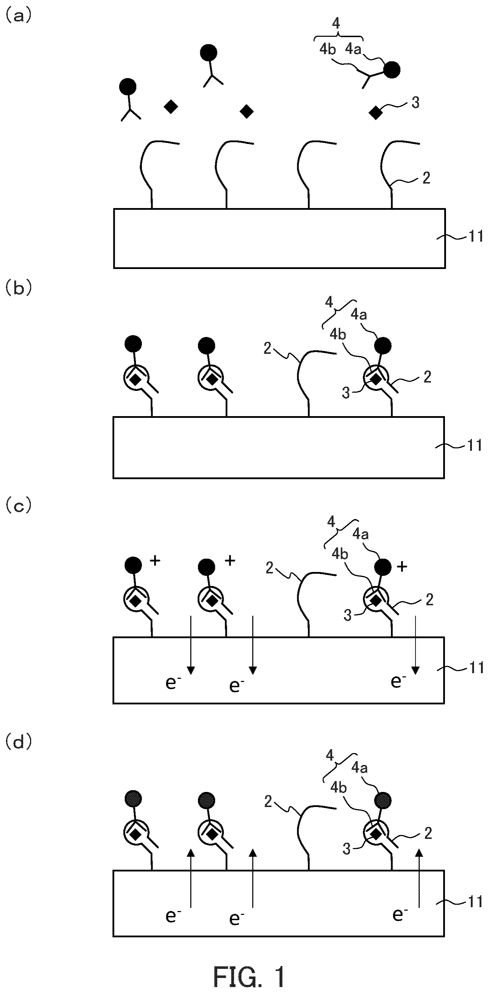

Hereinafter, an example of the electrochemical analysis method of the invention will be described more specifically with reference to FIGs. 1 to 5. The present embodiment is merely an example of the electrochemical analysis method, and the electrochemical analysis method is not limited thereto.

### (First Analysis Method)

The first analysis method will be described with reference to FIG. 1. First, a working electrode 11 on which immobilized conjugates 2 are immobilized is prepared as an electrode section. After immobilizing the immobilized conjugates 2 on the working electrode 11, a surface of the working electrode 11 is cleaned as necessary. Next, labeled conjugates 4 are prepared. A test solution (sample to be analyzed) containing analytes 3, and the labeled conjugates 4 are supplied to the surface of the working electrode 11 ((a) in FIG. 1), and the labeled conjugates 4 are immobilized on the working electrode 11 ((b) in FIG. 1). The analytes 3 and the labeled conjugates 4 can be supplied to the surface of the working electrode 11 in any manner, such as dropping a solution onto the surface of the working electrode 11, or immersing the working electrode 11 in the solution. Here, a conjugate of at least one of the immobilized conjugate 2 or the labeled conjugate 4 is an aptamer. In the present example, an aptamer is used as the conjugate of the immobilized conjugate 2, and an antibody (protein) is used as the conjugate of the labeled conjugate 4 (conjugate 4b). The analyte 3 may be a biomarker, in particular, secretory immunoglobulin A (sIgA).

Next, the potential of the working electrode 11 is controlled to electrochemically oxidize metal particles 4a ((c) in FIG. 1C). For example, the potential of the working electrode 11 with respect to the reference electrode is held for a predetermined time at potential at which the metal particles 4a are electrochemically oxidized. The operation of holding the potential for a predetermined time is a preferrable method that can sufficiently oxidize the metal particles. When the holding time is, for example, 1 second or more, the metal particles can be sufficiently oxidized, and thus the detection sensitivity can be reliably improved. For example, even when the holding time is 100 seconds or more, the obtained current value hardly changes. Therefore, the holding time is preferably 1 second or more and 100 seconds or less. The holding time of the potential is more preferable within a range of 40 seconds or more and 100 seconds or less.

For applying potential at which the metal particles 4a are electrochemically oxidized to the working electrode 11, for example, the potential of the working electrode 11 may be changed with the lapse of time, through cyclic voltammetry or the like. When changing the potential of the working electrode 11 with the lapse of time, it is preferable to change the potential of the electrode 11 within a range where the metal particles 4a can be oxidized (e.g., +1 to +2V with respect to a silver/silver chloride reference electrode). Further, for oxidizing the metal particles 4a, the potential at which the metal particles 4a be electrochemically oxidized may be applied to the working electrode 11 multiple times.

Here, for sufficiently oxidizing the metal particles 4a, it is necessary to pay attention to application of the optimum charge amount depending on the quantity of the metal particles 4a. Since the charge amount is a value obtained by integrating the current, when the potential applied to the working electrode 11 is relatively low, this potential needs to be applied for a long time to sufficiently oxidize the metal particles. In contrast, when the potential applied to the working electrode 11 is relatively high, the time required for sufficiently oxidizing the metal particles 4a can be shorter.

Through the above operation, the metal particles 4a collected in the vicinity of the surface of the working electrode 11 are completely oxidized. In the case of using metal fine particles with the particle diameter of 10 to 60 nm as the metal particles 4a, for electrochemically oxidizing the metal fine particles, it is preferable to set the potential of the working electrode to +1.2 to +1.6 V with respect to the silver/silver chloride reference electrode in a 0.1 to 0.5 N hydrochloric acid solution. During the electrochemical oxidation of the metal particles 4a, a counter electrode and a reference electrode are also in contact with the solution (not shown).

The potential of the working electrode 11 needs to be appropriately set to an optimum value depending on the type of the metal particles 4a to be used, and for example, preferably be +1 to +2 V and more preferably be +1.2 to +1.6 V, with respect to the silver/silver chloride reference electrode. By setting the potential of the working electrode 11 within the above range, the metal particles 4a collected in the vicinity of the surface of the working electrode 11 can be completely oxidized and eluted, and thus the sensitivity of the detection on the analyte 3 can be reliably improved. When the potential of the working electrode 11 is below the above range, the peak of the reduction current may not appear at the time of measurement. On the other hand, when the potential exceeds the above range, diffusion may occur due to migration of the oxidized metal particles 4a, causing the concentration of the oxides in the vicinity of the working electrode 11 to decrease, and resulting in decrease in the reduction current peak.

Next, the oxidized metal particles 4a are electrochemically reduced by controlling the potential of the working electrode 11 ((d) in FIG. 1). A current value generated by the reduction is measured, and the presence or the concentration of the analyte 3 can be determined based on the current value. Specifically, for example, the potential of the working electrode 11 is changed in a negative direction, and the current change associated with the potential change is measured. In association with the change of the electrode potential in a negative direction, the metal oxidized and eluted by the above-described potential control is reduced, thereby causing reduction current to flow, and this reduction current is measured. The intensity of the reduction current increases with a larger amount of the analytes in the sample, and a larger amount of metal particles collected in the vicinity of the working electrode 11. Based on this, quantification or detection of the analyte can be realized. The concentration of the analyte can be determined by, for example, determining a relationship between the reduction current value and an analyte with a known concentration in advance, and comparing the measured reduction current values. Also, the presence of the analyte in the sample to be analyzed can be detected from the obtained reduction current value.

A solution used for the potential control of the working electrode 11 and the electrochemical measurement is preferably, for example, acidic solutions, which can electrochemically oxidize the metal particles 4a easily. The acidic solution may be appropriately selected depending on the type of the metal particles 4a and the like, and may be, for example, an aqueous solution containing hydrochloric acid, nitric acid, acetic acid, phosphoric acid, citric acid, sulfuric acid, or the like. From the perspective of the ease of the electrochemical oxidation of the metal particles 4a, it is preferable to use a hydrochloric acid aqueous solution with the normality of 0.05 to 2 N , and it is more preferable to use a hydrochloric acid aqueous solution with the normality of 0.1 to 0.5 N. In the electrochemical oxidization of the metal particles 4a, for example, the potential of the working electrode 11 with respect to the silver/silver chloride reference electrode is preferably held at +1.2 to +1.6 V for 40 seconds or more and 100 seconds or less in a hydrochloric acid aqueous solution with the normality of 0.1 to 0.5 N.

On the other hand, the solution used for the potential control of the working electrode 11 and the electrochemical measurement may be other than an acid solution, and may be a neutral solution containing chlorine. Using a neutral solution containing chlorine results in larger current change compared to the use of an acidic solution, and consequently, higher sensitivity measurement is achieved. Further, when using an acidic solution, the peak shape may become asymmetric, like for example, the tail of the reduction peak on the low potential side may rise, or for example, noise may generates near 0.1 V. In contrast, when using a neutral solution containing chlorine, the tail of the reduction peak becomes flat and the noise generation can be reduced, making detection of the reduction peak intensity simpler. Furthermore, it is possible to avoid the use of solutions that are difficult to handle, such as acidic or alkaline solutions, and thus achieve safe and simple measurement operation. As neutral solutions containing chlorine, the above-mentioned effect can be obtained when using, for example, KCl, NaCl, LiCl, or the like.

Examples of a method for measuring the current generated when electrochemically reducing oxidized metal include voltammetry such as differential pulse voltammetry and cyclic voltammetry, amperometry, and chronometry.

In the first analysis method as described above, metal particles are collected in the vicinity of the surface of the working electrode, and the reduction peak current derived from the metal particles contained in the labeled conjugate is measured. Thus, the analyte in the sample to be analyzed can be measured in a simple manner, and with high sensitivity.

### (Second Analysis Method)

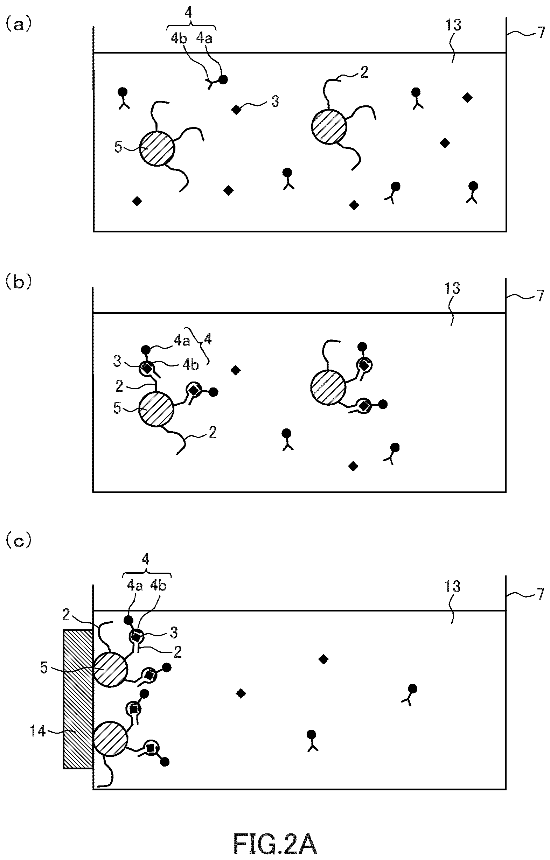

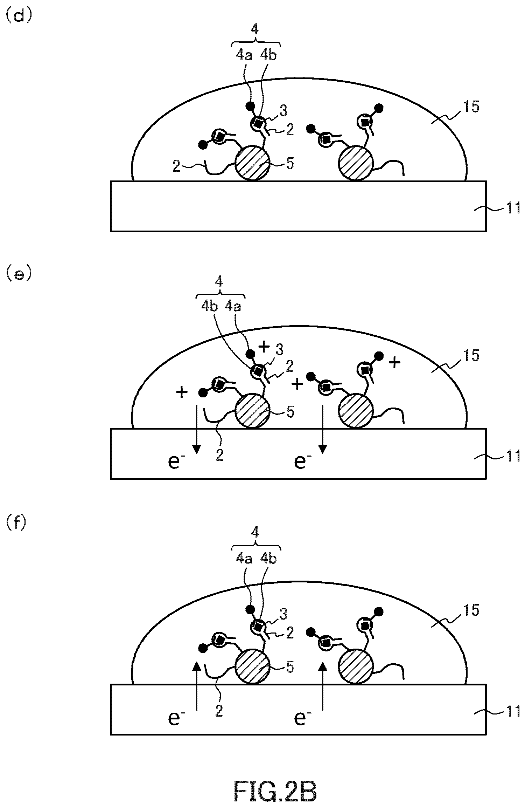

A second analysis method differs from the first analysis method in a specific process for collecting the metal particles in an amount corresponding to the analytes in the sample to be analyzed, in the vicinity of the surface of the working electrode. The present analysis method differs in that it realizes collecting the metal particles in an amount corresponding to the analytes in the sample to be analyzed in the vicinity of the surface of the working electrode by collecting magnetic particles on the surface of the working electrode. Hereinafter, the second analysis method will be described with reference to FIGs. 2A and 2B. In the following description of each analysis method, description overlapping with the first analysis method described above will be abbreviated.

First, an immobilized conjugate 2 is labeled with a magnetic particle 5. In other words, the immobilized conjugate 2 is immobilized onto a surface of the magnetic particle 5. Further, labeled conjugates 4 similar to that used in the first analysis method are prepared ( (a) in FIG. 2A).

Next, a reaction solution 13 is prepared in a predetermined container 7. The reaction solution 13 is a mixture of the magnetic particles 5 on which the immobilized conjugates 2 are respectively immobilized, the labeled conjugates 4, and a sample to be analyzed containing analytes 3 at an unknown concentration. By incubating this mixture for a predetermined time, the labeled conjugate 4 respectively bind to the magnetic particles 5 via the analytes 3 ((b) in FIG. 2A).

Next, the magnetic particles 5 are separated from the reaction solution 13 using magnet 14 ((c) in FIG. 2A). Thereafter, the separated magnetic particles 5 are suspended in a solution for electrochemical measurement.

Next, the metal particles 4a are collected in the vicinity of a surface of a working electrode 11 ((d) in FIG. 2B). Specifically, the magnetic particles 5 to which the conjugates are respectively bound are suspended in the solution for electrochemical measurement 15, and the suspension is supplied to the surface of the working electrode 11 by a method such as dropping or the like. Thereafter, for example, the supplied suspension is allowed to stand for a predetermined time to precipitate the magnetic particles 5, thereby obtaining a state where the metal particles 4a are collected in the vicinity of the surface of the working electrode 11. Alternatively, placing magnet on a back surface of the working electrode 11 to magnetically adsorb the magnetic particles 5 to the surface of the working electrode 11 can shorten the time for collecting the metal particles 4a bound to the magnetic particles 5 in the vicinity of the working electrode 11 (not shown).

The subsequent processes are the same as those of the first analysis method described above. That is, the metal particles 4a are electrochemically oxidized. Preferably, the potential of the working electrode 11 with respect to the reference electrode is held for a predetermined time at potential where the metal particles 4a are electrochemically oxidized. As a result, the metal particles 4a collected in the vicinity of the surface of the working electrode 11 are completely oxidized ((e) in FIG. 2B). At this time, the counter electrode and reference electrode are also in contact with the solution (not shown).

After the electrochemical oxidation, the presence or the concentration of the analyte is measured based on the peak current value generated when the oxidized metal is reduced ((f) in FIG. 2B). Specifically, the potential of the working electrode 11 is changed in a negative direction, and the current change associated with the potential change is measured. In this manner, the analyte concentration and the presence of the analyte can be determined in the same manner as in the first analysis method.

Additionally, in the present analysis method, the analyte 3 is captured by immobilizing the conjugate 2 on the magnetic particle 5 that can be suspended in the reaction solution. Therefore, the reaction efficiency between the analytes 3 and the labeled conjugates 4 can be increased compared to when immobilizing the conjugates 2 on the surface of the working electrode 11.

Further, the use of the magnetic particles 5 that can be magnetically separated can reduce the amount of the solution for electrochemical measurement 15 required for suspending the magnetic particles 5. That is, the magnetic particles 5 (metal particles 4a) can be present at a high concentration in the solution for electrochemical measurement 15, and thus the detection sensitivity can be further improved.

### (Third Analysis Method)

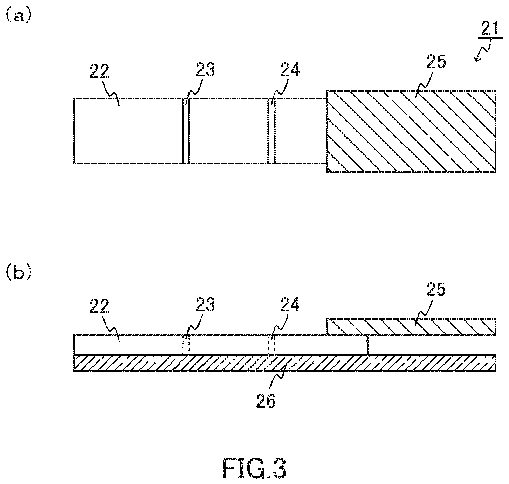

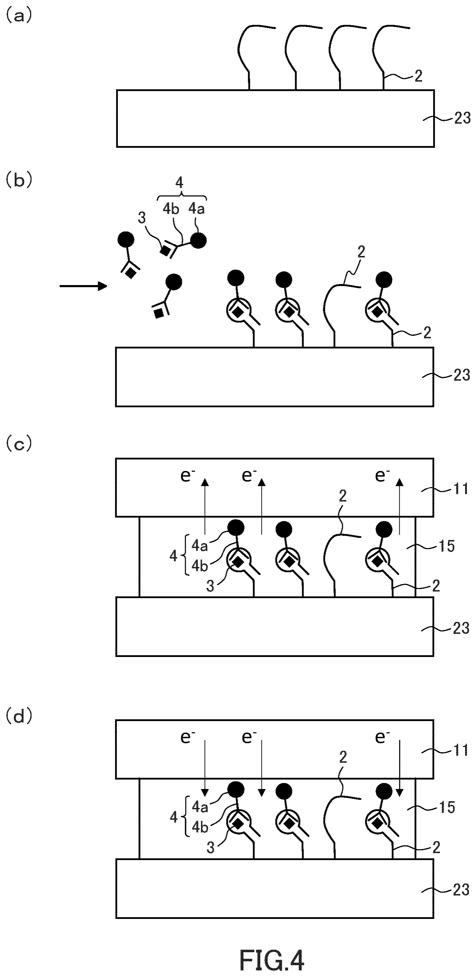

A third analysis method differs from the first analysis method in a specific process for collecting the metal particles in an amount corresponding to the analytes in the sample to be analyzed, in the vicinity of the surface of the working electrode. That is, in the third analysis method, the metal particles in an amount corresponding to the analytes in the sample to be analyzed can be collected in the vicinity of the surface of the working electrode by preparing the immobilized conjugates and the labeled conjugates, immobilizing the immobilized conjugates on the determination region included in the lateral flow analysis section, labeling the labeled conjugate with the metal particle, developing the sample to be analyzed and the labeled conjugates in the development region, and then making the development region face the surface of the working electrode. Hereinafter, an example of application to a lateral flow method (immunochromatography method) will be described with reference to FIG. 3 and FIG. 4.

A structure of a strip used for the lateral flow analysis is not particularly limited, and for example, a lateral flow strip 21 as shown in FIG. 3 can be used. The lateral flow strip 21 includes, for example, a strip-shaped membrane 22 made of nitrocellulose, an absorbent pad 25 joined to a downstream side of the membrane 22, and a backing sheet 26 disposed on a back surface of the membrane 22. As shown in FIG. 3 and (a) in FIG. 4, immobilized conjugates 2 are immobilized in a predetermined region of the surface of the membrane 22 to form a determination region (immobilization region) 23. On the surface of the membrane 22 at the downstream of the determination region 23, labeled conjugates 4 respectively labeled with a metal particle 4a are disposed to form a control region 24.

To detect the analyte 3 in the sample to be analyzed, first, the sample to be analyzed is developed in the same manner as in the conventional lateral flow method. That is, the sample to be analyzed and the labeled conjugates 4 are mixed, absorbed at one end (the left end in FIG. 3) of the lateral flow strip 21, and developed utilizing capillary phenomenon. When the analyte 3 is present in the sample to be analyzed, the immobilized conjugate 2 and the labeled conjugate 4 bind to the analyte 3 in a sandwich manner, and as a result, the metal particles 4a in an amount corresponding to the analytes 3 are captured by the determination region 23 ((b) in FIG. 4). The color development by the labeled conjugates 4 captured at the control region 24 indicates the completion of the development.

Next, at least the determination region 23 of the membrane 22 and the working electrode 11 are superimposed. Thus, the metal particles 4a collected in the determination region 23 are brought close to the surface of the working electrode 11 and collected thereto ((c) in FIG. 4). In order to reliably reduce the distance between the metal particles 4a and the working electrode 11, pressurization may be performed after superimposing at least the determination region 23 of the membrane 22 and the working electrode 11. The pressurization may be performed lightly to the extent where the surface of the membrane 22 and the surface of the working electrode 11 reliably come into contact with each other.

A gap between at least the determination region 23 of the membrane 22 and the working electrode 11 is to be filled with the solution for electrochemical measurement 15. At this time, although not shown, the counter electrode and reference electrode are to be also in contact with the solution 15.

The subsequent processes are the same as those of the first analysis method described above. That is, the metal particles 4a are electrochemically oxidized. Preferably, the potential of the working electrode 11 with respect to the reference electrode is held for a predetermined time at potential where the metal particles 4a are electrochemically oxidized. As a result, the metal particles 4a collected in the vicinity of the working electrode 11 are completely oxidized.

After the electrochemical oxidation, on the basis of the peak current value generated when the oxidized metal is reduced, the presence or the concentration of the analyte is measured ((d) in FIG. 4). Specifically, the potential of the working electrode 11 is changed in a negative direction, and the current change associated with the potential change is measured. In this manner, the concentration of the analyte can be obtained in the same manner as in the first example. Also, the presence of the analyte in the sample to be analyzed can be detected from the obtained reduction current value. Furthermore, high-sensitivity quantitative analysis can be realized without impairing simplicity of the lateral flow analysis.

### (Fourth Analysis Method)

Hereinafter, a fourth analysis method will be described. In the first analysis method described above, a primary antibody as a first binding substance is immobilized only on the working electrode, and for example, antigen-antibody reaction is performed using only the working electrode as a reaction field. However, in the case of using a planar device in which the working electrode, the counter electrode, and the reference electrode are formed on the same substrate, for example by printing, the area of the working electrode as the reaction field is limited by the size of the device itself, the area of the counter electrode, the area of the reference electrode, and the like. Accordingly, there is a limit to the improvement of the sensitivity in the measurement method described in the first analysis method.

Hence, in the present analysis method, both the working electrode and at least the counter electrode are used as reaction fields for antigen-antibody reaction and the like, and at least the metal particles collected in the vicinity of the surface of the counter electrode are oxidized and eluted, electrochemically migrated to the working electrode, and deposited on the surface of the working electrode. Thereafter, in the same manner as in the first example embodiment, the metal particles collected in the vicinity of the surface of the working electrode are electrochemically oxidized. This enables the metal particles collected in regions other than the working electrode to also be subject to the electrochemical measurement, meaning more metal particles are efficiently collected on the surface of the working electrode, realizing further improvement in detection sensitivity.

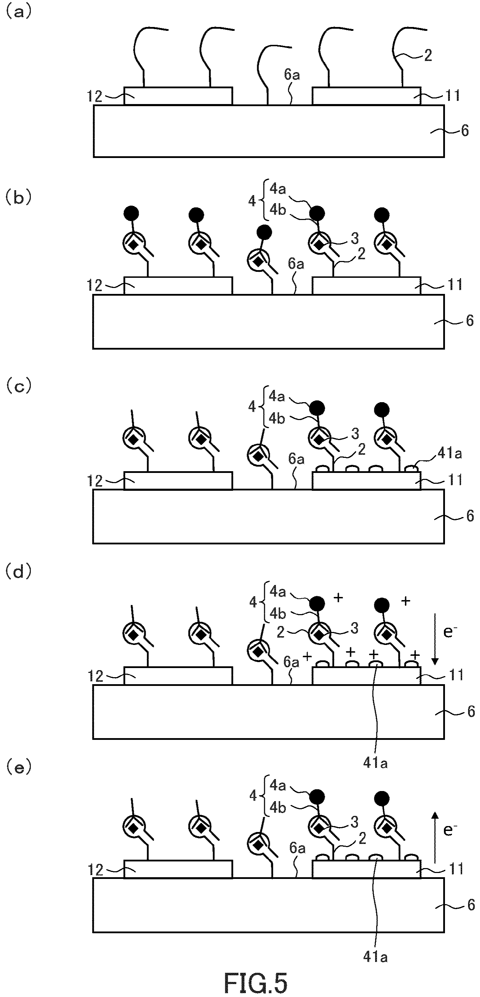

Specifically, first, as shown in (a) in FIG. 5, a planar electrode device in which a working electrode 11, a counter electrode 12, and a reference electrode (not shown) are formed on a same substrate 6 is prepared, and immobilized conjugates 2 are immobilized on both the working electrode 11 and the counter electrode 12. The immobilized conjugates 2 are also immobilized on an inter-electrode region 6a among the substrate 6, sandwiched by the working electrode 11 and the counter electrode 12. By immobilizing the immobilized conjugates 2 also on the inter-electrode region 6a, even more sensitive detection can be achieved. The electrode device surface is blocked to prevent non-specific adsorption. Additionally, a labeled conjugate 4 labeled with a metal particle 4a is prepared. Note that, the working electrode, the counter electrode, and the reference electrode are not necessarily formed on the same substrate as long as being present at positions close to each other.

Next, a sample to be analyzed containing the labeled conjugates 4 and an unknown amount of the analytes 3 is supplied to the surfaces of the working electrode 11, the counter electrode 12, and the inter-electrode region 6a, and is brought into contact with the immobilized conjugates 2. Thus, the labeled conjugates 4 respectively bind to the immobilized conjugates 2 via the analytes 3, and the metal particles 4a corresponding to the concentration of the analytes 3 are collected in the vicinity of the surfaces of the working electrode 11, the counter electrode 12, and the inter-electrode region 6a ((b) in FIG. 5).

Next, after cleaning the surfaces of the working electrode 11, counter electrode 12, and inter-electrode region 6a as necessary, these surfaces are brought into contact with a solution for electrochemical measurement, and potential control is performed so that the counter electrode 12 has a positive potential with respect to the working electrode 11. As a result, the metal particles 4a collected in the vicinity of the surface of the counter electrode 12 are oxidized and eluted, and electrically migrate in the solution. The metal particles 4a collected in the vicinity of the surface of the inter-electrode region 6a located between the working electrode 11 and the counter electrode 12 also electrically migrate in the solution. When those metal particles 4a reach the surface of the working electrode 11, they deposit as metal 41a ((c) FIG. 5). As a result, all the metal particles 4a involved in the reaction are collected in the vicinity of the working electrode 11.

In order to elute at least the metal particles 4a in the vicinity of the surface of the counter electrode 12 and cause them to migrate and deposit on the surface of the working electrode 11, the potential of the counter electrode 12 with respect to the working electrode 11 needs to be potential at which the metal particles 4a are oxidized. Specifically, although it depends on the measurement solution to be used, for example, the potential of the counter electrode with respect to the working electrode 11 is preferably within a range of +1 to +2V. By setting the potential within this range, at least the metal particles 4a collected on the surface of the counter electrode 12 can be reliably eluted and cause them to migrate onto the working electrode 11. The potential of the counter electrode 12 with respect to the working electrode 11 may be held for a predetermined time at potential at which the metal particles 4a are oxidized, or may be changed with the lapse of time within the range of the potential at which the metal particles 4a are oxidized.

The subsequent processes are the same as those of the first analysis method described above. That is, the metal particles 4a collected in the vicinity of the surface of the working electrode 11 are electrochemically oxidized. As a result, the metal particles 4a collected in the vicinity of the working electrode 11 are completely oxidized ((d) in FIG. 5). At this time, the metal 41a deposited on the surface of the working electrode 11 is also oxidized. Next, peak current generated when reducing the oxidized metal is measured. On the basis of the measured peak current, the concentration or the like of the analyte is examined ((e) in FIG. 5). Specifically, for example, the potential of the working electrode 11 is changed in a negative direction, and the current change associated with the potential change is measured. In association with the change of the electrode potential in a negative direction, the metal oxidized (eluted) by the above-described potential control is reduced, thereby causing reduction current to flow, and this reduction current is measured. The concentration of the analyte can be determined by determining a relationship between the reduction current value and an analyte with a known concentration in advance, and comparing the measured reduction current values. Also, the presence of the analyte in the sample to be analyzed can be detected from the obtained reduction current value.

As described above, in the present analysis method, at least the counter electrode 12 other than the working electrode 11 is also used as a reaction field, and the metal particles 4a collected in the vicinity of the surface of the counter electrode 12 are transferred to the surface of the working electrode 11, so that the reduction current can be measured for all the metal particles 4a in the conjugates involved in the reaction, thereby realizing higher sensitivity compared to when only the surface of the working electrode 11 is used as a reaction field. In addition, the migration of the metal particles 4a collected on the surface of the counter electrode 12 to the surface of the working electrode 11 is achieved by simple operation of controlling the potential between the working electrode 11 and the counter electrode 12, eliminating the need for mechanical structures such as those for stirring the solution. Therefore, higher sensitivity can be realized by extremely simple operation, without changing the structure of the electrode device.

In the above description, as a method for collecting the metal particles in an amount corresponding to the amount of analytes, a method of collecting the metal particles in an amount corresponding to the amount of analytes in a sample to be analyzed using non-competitive reaction is exemplified. However, a method of collecting metal particles in an amount corresponding to the amount of analytes in a sample to be analyzed using competitive reaction may also be adopted.

### Examples

Next, examples of the invention will be described. However, the invention is not limited to the following examples.

### <Aptamer>

In the present example, an aptamer was used as an immobilized conjugate, and an antibody was used as a labeled conjugate. As the aptamer, a sIgA-binding nucleic acid molecule described in Japanese Patent No. 6795211 was used. As the antibody, Anti-IgA(α), Human, Goat-Poly (manufactured by Bethyl Laboratories, catalog number: A80-102A-1) was used.

### <Electrochemical Analysis Kit (Sensor)>

Aptamers as immobilized conjugates were immobilized on an electrode to prepare an electrochemical analysis kit (hereinafter, also referred to as a "sensor") for use in the present example. A planar printed electrode was used as the electrode.

### <Labeled Conjugate>

The antibody as the label conjugate was labeled with a gold nanoparticle (manufactured by BBI Solutions, product code: EMGC40).

### [Example 1]

In the present example, the prepared sensor was used to measure the sensor output for sIgA with known concentration.

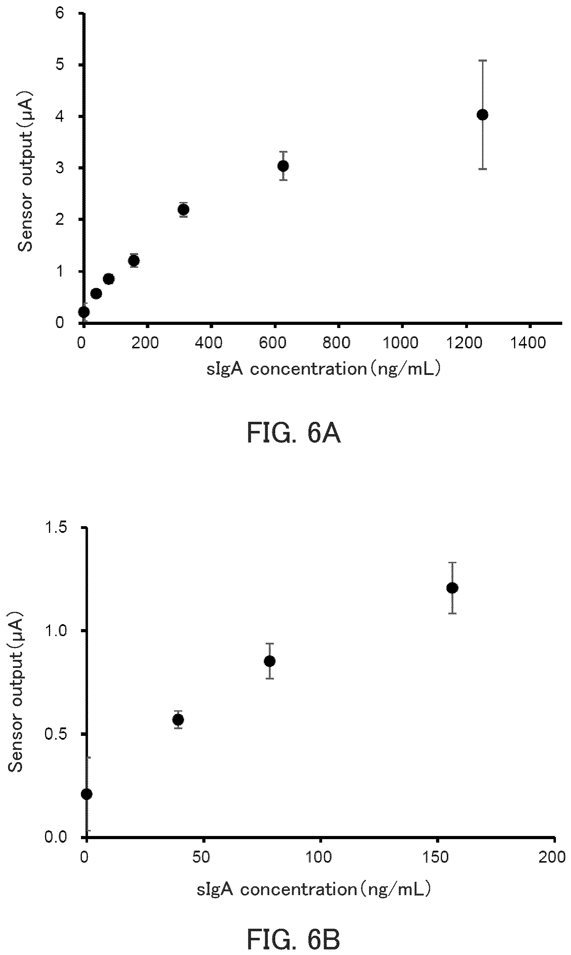

First, human sIgA (IgA (Secretory), Human, manufactured by MP Biomedicals, LLC, Cappel products, catalog number: #55905) was diluted with a sample dilute solution to be 40 to 1250 ng/mL. The sample dilute solution used was 1×PBS (10×PBS Buffer (-), manufactured by NIPPON GENE CO., LTD., catalog number: 314-90185) and 1% BSA (bovine serum albumin derived from bovine serum, manufactured by Sigma-Aldrich, catalog number: A7030). Next, 55 µL of the diluted sIgA was added dropwise to the prepared kit (sensor) and allowed to stand for 7 minutes. Then, 250 µL of a cleaning solution was added dropwise and allowed to stand for 3 minutes. The cleaning solution used was 2M NaCl (5M NaCl, manufactured by NACALAI TESQUE, INC., catalog number: 31334-51) and 0.05% Tween^{®} 20 (TWEEN^{®} 20, manufactured by Sigma-Aldrich, catalog number: P7949). Thereafter, voltage was applied to the sensor, and peak current at 100 to 650 mV was measured. The results are shown in FIGs. 6A and 6B. FIGs. 6A and 6B are graphs showing the relationship between the sIgA concentration and the sensor output. In FIGs. 6A and 6B, the horizontal axis represents the sIgA concentration (ng/mL), and the vertical axis represents the sensor output (µA).

### [Example 2]

In the present example, a commercially available saliva sample was used to determine the correlation between the sIgA concentration analysis results from the electrochemical analysis kit of the invention and the ELISA method.

First, instead of human sIgA, two types of 400-fold diluted human saliva (Saliva - Normal, Male Donor, Human, manufactured by Lee BioSolutions, Inc, catalog number: 991-05-M (hereinafter, also referred to as "M01"), and Saliva - Normal, Female Donor, Human, manufactured by Lee BioSolutions, Inc, catalog number: 991-05-F (hereinafter, also referred to as "F01")), and the M01 and the F01 respectively added with standard sIgA (IgA (Secretory), Human, manufactured by MP Biomedicals, LLC, Cappel products, catalog number: #55905) were used as samples. As the samples added with the standard sIgA, a total of seven types of samples: samples obtained by adding 25 ng, 50 ng, 100 ng, and 200 ng of sIgA to 400 µL of the 400-fold diluted M01, and samples obtained by adding 25 ng, 50 ng, and 100 ng of sIgA to 400 µL of the 400-fold diluted F01. By the same procedure as in Example 1 except that the samples used for analysis were changed, the sensor outputs of a total of nine types of samples were measured using the prepared sensor. Details of the M01 and the F01 are as shown in Table 1 below.

**[Table 1]**

| Sample number | Birth Year | Gender | Race |
|---|---|---|---|
| M01 | 1986 | Male | Caucasian |
| F01 | 1989 | Female | Caucasian |

Next, the same sample as that measured by the sensor was analyzed by an ELISA kit (ELISA Kit for Secretory Immunoglobulin A (sIgA), manufactured by CLOUD-CLONE CORP., catalog number: SEA641HU), and the absorbance was measured.

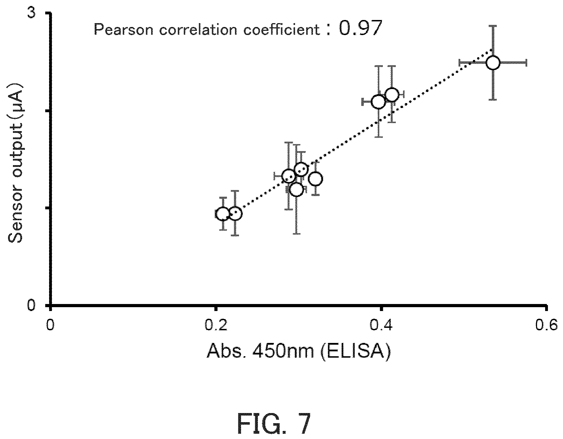

The results are shown in FIG. 7. FIG. 7 is a graph showing the relationship between the sensor output obtained from the electrochemical analysis kit of the invention and the absorbance at wavelength of 450 nm obtained by the ELISA method. In FIG. 7, the horizontal axis represents the absorbance, and the vertical axis represents the sensor output (nA) according to the electrochemical analysis kit of the invention.

### <Results>

As shown in FIG. 6, the electrochemical analysis kit of the invention was able to measure very low concentration of sIgA in the range of 40 to 1250 ng/mL.

Further, as shown in FIG. 7, there was a correlation between the absorbance measurement result by the ELISA method, which is widely adopted as a method for measuring trace substance concentrations, and the sensor output obtained from the electrochemical analysis kit of the invention (Pearson correlation coefficient: 0.97). In other words, it was found that the concentration analysis results from the electrochemical analysis kit of the invention were highly accurate.

While the invention has been particularly shown and described with reference to example embodiments thereof, the invention is not limited to these embodiments. It will be understood by those of ordinary skill in the art that various changes in form and details may be made therein without departing from the spirit and scope of the invention as defined by the claims.

### SUPPLEMENTARY NOTE

The whole or part of the example embodiments disclosed above can be described as, but not limited to, the following supplementary notes.

### (Supplementary Note 1)

An electrochemical analysis kit, including:
an electrode section,
an immobilized conjugate, and
a labeled conjugate, wherein
the electrode section includes a pair of electrodes,
the pair of electrodes includes:
   a working electrode, and
   a counter electrode,
   the immobilized conjugate is capable of specifically binding to an analyte, and of being immobilized at a specific site in a state of being bound to the analyte,
   the labeled conjugate is capable of specifically binding to the analyte, and is labeled with a metal particle,
   when the analyte is contained in a sample to be analyzed, the immobilized conjugate and the labeled conjugate bind to the analyte, so that the labeled conjugate be immobilized at the specific site,
   the specific site is a position where the metal particle can be oxidized by control of potential of the working electrode,
   after the oxidation of the metal particle, the metal particle is reduced by control of potential of the working electrode,
   current generated by the reduction can be measured with the pair of electrodes, and a conjugate of at least one of the immobilized conjugate or the labeled conjugate is an aptamer.

### (Supplementary Note 2)

The kit according to Supplementary Note 1, wherein
the working electrode and the counter electrode in the pair of electrodes are interchangeable with each other.

### (Supplementary Note 3)

The kit according to Supplementary Note 1 or 2, wherein
the electrode section further includes a reference electrode.

### (Supplementary Note 4)

The kit according to any one of Supplementary Notes 1 to 3, further including
a lateral flow analysis section, wherein
the lateral flow analysis section includes:
   a development region;
   a sample supply region, and
   a determination region,
   the sample supply region is arranged at one end of the development region,
   the determination region is arranged at opposite side of the development region from the position where the sample supply region is arranged,
   the electrode section and the immobilized conjugate are arranged at the determination region,
   the sample supply region is configured to allow the analyte and the labeled conjugate to be supplied thereto, and
   the development region is configured to allow movement of the sample and the labeled aptamer to the determination region by capillary phenomenon.

### (Supplementary Note 5)

The kit according to any one of Supplementary Notes 1 to 4, wherein
the sample to be analyzed is human saliva, and
the analyte is a biomarker.

### (Supplementary Note 6)

The kit according to Supplementary Note 5, wherein
the biomarker is secretory immunoglobulin A (sIgA).

### (Supplementary Note 7)

An electrochemical analysis system, including:
an electrochemical analysis apparatus, and
an electrochemical analysis kit, wherein
the electrochemical analysis kit is the kit according to any one of Supplementary Notes 1 to 6, and
the electrochemical analysis apparatus is capable of applying voltage to the electrode and measuring current of the electrode.

### (Supplementary Note 8)

An electrochemical analysis method using the kit according to any one of Supplementary Notes 1 to 6, including:
oxidizing the metal particle bound to the labeled conjugate by controlling the potential of the working electrode,
reducing the metal particle by controlling the potential of the working electrode, and
qualitatively or quantitatively analyzing the analyte by measuring the current generated by the reduction with the pair of electrodes.

### Industrial Applicability

The invention realizes, for example, accurate detection of the test substance. In addition, the use of an aptamer allows better storage stability, cost-effectiveness, synthesizability, and the like, compared to the use of an antibody. The fields to which the invention can be applied are not limited. The invention can be applied to a wide range of fields using electrochemical analysis kits. For example, the invention can be considered as an extremely useful tool in fields such as medicine, nursing care, healthcare, and the like.

### Reference Signs List

- 11:: working electrode
- 12:: counter electrode
- 13:: reaction solution
- 14:: magnet
- 15:: solution (solution for electrochemical measurement)
- 2:: immobilized conjugate
- 21:: lateral flow strip
- 22:: membrane
- 23:: determination region (immobilization region)
- 24:: control region
- 25:: absorbent pad
- 3:: analyte
- 4:: labeled conjugate
- 4a:: metal particle
- 4b:: conjugate (conjugate of labeled conjugate 4)
- 41a:: metal
- 5:: magnetic particle
- 6:: substrate
- 6a:: inter-electrode region
- 7:: container

## Claims

1. An electrochemical analysis kit, comprising:
an electrode section,
an immobilized conjugate, and
a labeled conjugate, wherein
the electrode section includes a pair of electrodes,
the pair of electrodes includes:
a working electrode, and
a counter electrode,
the immobilized conjugate is capable of specifically binding to an analyte, and of being immobilized at a specific site in a state of being bound to the analyte,
the labeled conjugate is capable of specifically binding to the analyte, and is labeled with a metal particle,
when the analyte is contained in a sample to be analyzed, the immobilized conjugate and the labeled conjugate bind to the analyte, so that the labeled conjugate be immobilized at the specific site,
the specific site is a position where the metal particle can be oxidized by control of potential of the working electrode,
after the oxidation of the metal particle, the metal particle is reduced by control of potential of the working electrode,
current generated by the reduction can be measured with the pair of electrodes, and
a conjugate of at least one of the immobilized conjugate or the labeled conjugate is an aptamer.

2. The kit according to Claim 1, wherein
the working electrode and the counter electrode in the pair of electrodes are interchangeable with each other.

3. The kit according to Claim 1, wherein
the electrode section further includes a reference electrode.

4. The kit according to Claim 1, further comprising
a lateral flow analysis section, wherein
the lateral flow analysis section includes:
a development region;
a sample supply region, and
a determination region,
the sample supply region is arranged at one end of the development region,
the determination region is arranged at opposite side of the development region from the position where the sample supply region is arranged,
the electrode section and the immobilized conjugate are arranged at the determination region,
the sample supply region is configured to allow the analyte and the labeled conjugate to be supplied thereto, and
the development region is configured to allow movement of the sample and the labeled aptamer to the determination region by capillary phenomenon.

5. The kit according to Claim 1, wherein
the sample to be analyzed is human saliva, and
the analyte is a biomarker.

6. The kit according to Claim 5, wherein
the biomarker is secretory immunoglobulin A (sIgA).

7. An electrochemical analysis system, comprising:
an electrochemical analysis apparatus, and
an electrochemical analysis kit, wherein
the electrochemical analysis kit is the kit according to any one of Claims 1 to 6, and
the electrochemical analysis apparatus is capable of applying voltage to the electrode and measuring current of the electrode.

8. An electrochemical analysis method using the kit according to any one of Claims 1 to 6, comprising:
oxidizing the metal particle bound to the labeled conjugate by controlling the potential of the working electrode,
reducing the metal particle by controlling the potential of the working electrode, and
qualitatively or quantitatively analyzing the analyte by measuring the current generated by the reduction with the pair of electrodes.
